# EUROPÄISCHE PATENTSCHRIFT

(11) **EP 0 633 017 B1**
(45) Veröffentlichungstag und Bekanntmachung des Hinweises auf die Patenterteilung: **10.02.1999**
(21) Anmeldenummer: 94108203.4
(22) Anmeldetag: 28.05.1994
(51) Int. Cl.: A61K 7/48, A61K 31/195

(54) **Kosmetische und dermatologische Zubereitungen mit einem Gehalt an Delta-Aminolävulinsäure**
Cosmetic and dermatologic compositions containing delta-aminolevulinic acid
Compositions cosmétiques et dermatologiques contenant de l'acide delta-aminolevulinique

(30) Priorität: 24.06.1993 DE 4320871
(43) Veröffentlichungstag der Anmeldung: 11.01.1995
(73) Patentinhaber: Beiersdorf Aktiengesellschaft, 20245 Hamburg (DE)
(72) Erfinder: Uhlmann, Beate, Dr., D-22453 Hamburg (DE); Gers-Barlag, Heinrich, Dr., D-25495 Kummerfeld (DE); Mann, Tobias, D-22179 Hamburg (DE); Sauermann, Gerhard, Dr., D-24649 Wiemersdorf (DE)
(74) Vertreter: Dinné, Erlend

(56) Entgegenhaltungen:
- WO-A-91/01727
- S.T.N., Datenbank Lifferant, Karlsruhe, DE, Datenbank Chemical Abstract, Band 117, N 208111 ziehe die Zusammenfassung
- S.T.N., Datenbank Lifferant, Karlsruhe, DE, Datenbank Medline, AN 94180707 ziehe die Zusammenfassung
- S.T.N., Datenbank Lifferant, Karlsruhe, DE, Datenbank Medline, AN 91038594 ziehe die Zusammenfassung

## Beschreibung

Die vorliegende Erfindung betrifft kosmetische und dermatologische Zubereitungen. Die Erfindung betrifft bevorzugt solche Zubereitungen, welche die Folgeschäden einer Lichtexposition, insbesondere einer Ultraviolettlichtexposition, lindern und solche Zubereitungen, welche vor einer Lichtexposition, insbesondere einer Ultraviolettlichtexposition, angewandt, solchen Folgeschäden zuvorkommen, also präventiv wirken.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch Filtersubsubstanzen für die Strahlung im Bereich zwischen etwa 320 nm und etwa 400 nm, den sogenannten UVA-Bereich, sind wichtig, da auch diese Strahlung Schäden hervorrufen kann. So führt UVA-Strahlung zu einer Schädigung der elastischen und kollagenen Fasern des Bindegewebes, was die Haut vorzeitig altern läßt und als Ursache zahlreicher phototoxischer und photoallergischer Reaktionen zu sehen ist. Ferner kann der schädigende Einfluß der UVB-Strahlung durch UVA-Strahlung verstärkt werden.

Zum Schutz gegen die Strahlen des UVA-Bereichs werden im wesentlichen gewisse Derivate des Dibenzoylmethans verwendet.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Ferner kann bei UV-Bestrahlung auch Singulettsauerstoff, ein nichtradikalischer angeregter Zustand des Sauerstoffmoleküls auftreten, ebenso kurzlebige Epoxide und viele Andere. Singulettsauerstoff zum Beispiel zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Um diesen Reaktionen vorzubeugen, können kosmetischen bzw. dermatologischen Formulierungen zusätzlich Antioxidantien und/oder Radikalfänger einverleibt werden.

Es ist schließlich bekannt, daß bestimmte Lichtdermatosen durch gewisse Emulgatoren, aber auch durch verschiedene Fette, und gleichzeitige Exposition von Sonnenlicht ausgelöst werden. Solche Lichtdermatosen werden auch "Mallorca-Akne"genannt.

Die teilweise vorstehend genannten Verbindungen, welche als Lichtschutzmittel für kosmetische und dermatologische Lichtschutzformulierungen eingesetzt werden, zeichnen sich an sich durch gute Lichtschutzwirkung aus. Sie haben jedoch den Nachteil, daß es bisweilen schwierig ist, sie in befriedigender Weise solchen Formulierungen einzuverleiben.

Es ist bereits vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Aufgabe der vorliegenden Erfindung war somit, kosmetische und dermatologische Wirkstoffe und Zubereitungen, solche Wirkstoffe enthaltend, zur Verfügung zu stellen, welche einen zuverlässigen Schutz vor Ultraviolettstrahlung gewährleisten bzw. Folgeschäden der UV-Expositionen vorbeugen, vorhandene Folgeschäden lindern oder diese gänzlich beseitigen.

Insbesondere war es eine Aufgabe der vorliegenden Erfindung, kosmetische und dermatologische Wirkstoffe und Zubereitungen, solche Wirkstoffe enthaltend, zur Verfügung zu stellen, welche der durch UV-Exposition hervorgerufenen oder verstärkten Hautalterung entgegenwirken.

Schließlich war es eine Aufgabe der vorliegenden Erfindung, kosmetische und dermatologische Wirkstoffe und Zubereitungen, solche Wirkstoffe enthaltend, zur Verfügung zu stellen, welche der sogenannten Mallorca-Akne entgegenwirken. Es war für den Fachmann nicht vorauszusehen, und darin liegt die Lösung dieser Aufgaben begründet, daß die Verwendung von δ-Aminolävulinsäure zur Herstellung kosmetischer oder dermatologischer Zubereitungen zum Schutze der Haut gegen schädlichen Einfluß von ultraviolettem Licht, ferner: kosmetische oder dermatologische Zubereitungen, enthaltend Wirkstoffkombinationen aus δ-Aminolävulinsäure und Antioxidantien, ferner: kosmetische oder dermatologische Zubereitungen, enthaltend Wirkstoffkombinationen aus δ-Aminolävulinsäure und einer oder mehreren Substanzen, die UV-Strahlung im UVA- oder UVB-Bereich absorbieren, schließlich: ein kosmetisches Verfahren zum Schutze der Haut vor UVA- und UVB-Strahlung, dadurch gekennzeichnet, daß man eine kosmetische Zubereitung, welche δ-Aminolävulinsäure enthält, in ausreichender Menge auf die Haut aufbringt, den Mißständen des Standes der Technik abhelfen.

δ-Aminolävulinsäure (auch δ-ALA, 5-Amino-4-oxo-pentansäure, δ-Amino-γ-keto-valeriansäure genannt) zeichnet sich durch folgende Struktur aus:

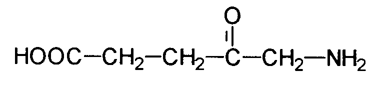

Die δ-Aminolävulinsäure ist ein Zwischenprodukt der Biosynthese der Porphyrine und entsteht in einem Nebenwege des Citronensäurezyklus.

B.Ortel, A.Tanew und H.Hönigsmann (J.Photochem. Photobiol. B: Biol., 17 (1993), S. 273 - 278) beschreiben, daß die externe Verabreichung von δ-Aminolävulinsäure an Tumorzellen die Biosynthese von Porphyrinen induziert. Die durch die erhöhte Konzentration von Porphyrinen gesteigerte Photosensitivität ermöglicht es, durch Bestrahlung mit Licht geeigneter Wellenlänge, diese Tumorzellen abzutöten.

Die WO-A-91/01727 offenbart Zubereitungen mit einem Gehalt an δ-Aminolävulinsäure zur Behandlung bösartiger Hautläsionen.

Der Fachmann konnte aber durch Kenntnis dieser Schriften nicht in die Richtung der hiermit vorgelegten Erfindung geleitet werden.

Besonders vorteilhafte Zubereitungen werden ferner erhalten, wenn δ-Aminolävulinsäure mit Antioxidantien kombiniert wird.

Die erfindungsgemäßen Antioxidantien können vorteilhaft aus der Gruppe der üblichen kosmetischen und dermatologischen Antioxidantien gewählt werden, insbesondere aus der Gruppe bestehend aus Tocopherolen und deren Derivaten, besonders α-Tocopherol bzw. α-Tocopherylestern, insbesondere α-Tocopherylacetat, ferner Sesamöl, Gallensäurederivaten wie Methyl-, Ethyl-, Propyl-, Amyl-, Butyl- und Laurylgallat, dem Konyferylbenzoat des Benzoeharzes, Nordihydroguajakharzsäure, Nordihydroguajaretsäure, Butylhydroxyanisol, Butylhydroxytoluol, Ascorbinsäure, Citronensäure, Phosphorsäure, Lecithin, Trihydroxybutyrophenon, Carotinen, Vitamin-A und dessen Derivaten, insbesondere Retinylpalmitat, Ascorbinsäure, Ascorbylpalmitat, Dilaurylthiodipropionat, Distearylthiodipropionat, Monoisopropylcitrat, Thiodipropionsäure und EDTA sowie EDTA-Derivaten.

Bevorzugt ist, die erfindungsgemäßen Antioxidantien aus der Gruppe der Tocopherole und deren Derivaten zu wählen.

Die Tocopherole, auch Vitamin E genannt, leiten sich vom Grundkörper Tocol ((2-Methyl-2-(4,8,12-trimethyltridecyl)chroman-6-ol) ab und sind durch folgende Strukturen gekennzeichnet:

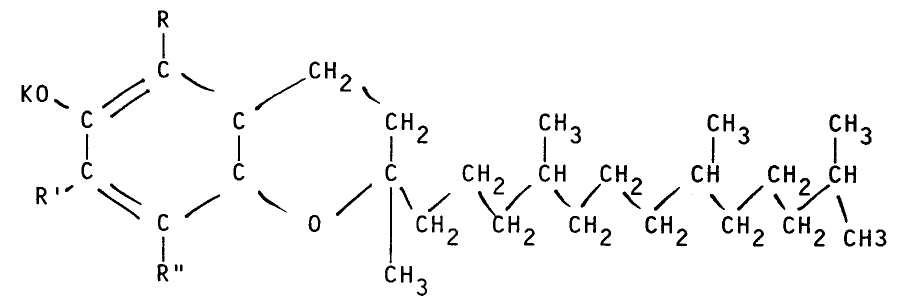

Dabei stellt K entweder H oder einen Acylrest dar, R, R' und R'' bedeuten unabhängig voneinander H oder eine Methylgruppe, z.B.: 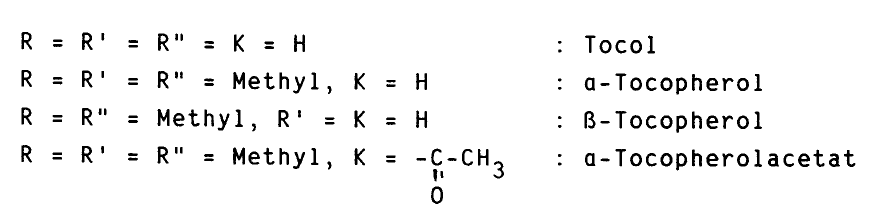 und weitere Varianten. In diesen Estern gilt für den Acylrest K: 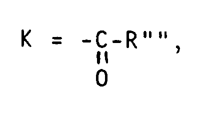 wobei R'''' einen Alkyl oder Alkenylrest mit 1 bis 21 Atomen darstellen kann. Besonders bevorzugt ist R'''' = Methyl.

Dem natürlich am häufigsten vorkommenden und bedeutendsten α-Tocopherol kommt die Konfiguration 2R,4'R,8'R zu. Es wird gelegentlich auch RRR-α-Tocopherol genannt.

Die erfindungsgemäß bevorzugten Tocopherolderivate sind das α-Tocopherol und seine Ester, insbesondere das α-Tocopherylacetat.

Es ist zwar aus US-4,144,325 und US-4,248,861 sowie aus zahlreichen anderen Dokumenten bekannt, Vitamin E in kosmetischen und dermatologischen Lichtschutzformulierungen einzusetzen. Die erfindungsgemäßen Kombinationen waren jedoch bisher unbekannt. Darüberhinaus gibt der Stand der Technik keinen Hinweis der in die Richtung der hiermit vorgelegten Erfindung weisen könnte.

In erstaunlicher Weise hat sich herausgestellt, daß die δ-Aminolävulinsäure in der Lage ist, den Prozessen entgegenzuwirken, die bei den durch Ultraviolettlicht hervorgerufenen oder von Ultraviolettlicht unterstützten Hautdegenerationen eine Rolle spielen.

Es war ferner nicht vorherzusehen gewesen, daß die δ-Aminolävulinsäure bzw. kosmetische oder dermatologische Zubereitungen mit einem wirksamen Gehalt an δ-Aminolävulinsäure bzw. die erfindungsgemäßen Kombinationen aus δ-Aminolävulinsäure sowie mindestens einer Substanz, gewählt aus der Gruppe der Antioxidantien, insbesondere der Tocopherole und Tocopherylester,
- besser gegen Schädigung durch UV-Strahlung schützen
- besser als Antioxidans wirken
- besser als Radikalfänger wirken
- besser die Bindung von schädlichen Photoprodukten an Lipide, DNS und Proteine verhindern
würden als die Präparate des Standes der Technik. Ferner war nicht vorherzusehen gewesen, daß die δ-Aminolävulinsäure, bzw. kosmetische oder dermatologische Zubereitungen mit einem wirksamen Gehalt an δ-Aminolävulinsäure bzw. die erfindungsgemäßen Kombinationen aus δ-Aminolävulinsäure sowie mindestens einer Substanz, gewählt aus der Gruppe der Antioxidantien, insbesondere der Tocopherole und Tocopherylester,
- für die Anwendung genügend hohe Stabilität aufweisen
- zu hautverträglichen Produkten führen
- nicht in die hauteigene Mikroorganismenflora eingreifen
- der lichtinduzierten Hautalterung entgegenwirken
- der Mallorca-Akne entgegenwirken würden.

Insbesondere war nicht vorherzusehen, daß die δ-Aminolävulinsäure, bzw. kosmetische oder dermatologische Zubereitungen mit einem wirksamen Gehalt an δ-Aminolävulinsäure bzw. die erfindungsgemäßen Kombinationen aus δ-Aminolävulinsäure sowie mindestens einer Substanz, gewählt aus der Gruppe der Antioxidantien, insbesondere der Tocopherole und Tocopherylester, sich durch eine ausgeprägte verzögerte Wirkung ("Retard-Wirkung") auszeichnen würden.

Darüber hinaus sind die die δ-Aminolävulinsäure, bzw. kosmetische oder dermatologische Zubereitungen mit einem wirksamen Gehalt an δ-Aminolävulinsäure bzw. die erfindungsgemäßen Kombinationen aus δ-Aminolävulinsäure sowie mindestens einer Substanz, gewählt aus der Gruppe der Antioxidantien, insbesondere der Tocopherole und Tocopherylester, in besonderem Maße zur Penetration in tiefergelegene Hautschichten geeignet, wo sie in vorteilhafter Weise ihre Wirkung entfalten können.

Weiterhin eignen sich die δ-Aminolävulinsäure, bzw. kosmetische oder dermatologische Zubereitungen mit einem wirksamen Gehalt an δ-Aminolävulinsäure bzw. die erfindungsgemäßen Kombinationen aus δ-Aminolavulinsäure sowie mindestens einer Substanz, gewählt aus der Gruppe der Antioxidantien, insbesondere der Tocopherole und Tocopherylester, erstaunlicherweise zur gezielten Prophylaxe und/oder Behandlung UV-induzierter Hautschäden.

Erfindungsgemäß ist auch die Verwendung der δ-Aminolävulinsäure, bzw. kosmetischer oder dermatologischer Zubereitungen mit einem wirksamen Gehalt an δ-Aminolävulinsäure bzw. der erfindungsgemäßen Kombinationen aus δ-Aminolävulinsäure sowie mindestens einer Substanz, gewählt aus der Gruppe der Antioxidantien, insbesondere der Tocopherole und Tocopherylester, zum Schutze der Haut gegen schädlichen Einfluß von ultraviolettem Licht.

In erstaunlicher Weise hat sich herausgestellt, daß die δ-Aminolävulinsäure, bzw. kosmetische oder dermatologische Zubereitungen mit einem wirksamen Gehalt an δ-Aminolävulinsäure bzw. die erfindungsgemäßen Kombinationen aus δ-Aminolävulinsäure sowie mindestens einer Substanz, gewählt aus der Gruppe der Antioxidantien, insbesondere der Tocopherole und Tocopherylester, in der Lage sind, photochemisch erzeugte Radikale abzufangen, vor photochemisch induzierten unkontrollierten Oxidationsprozessen zu schützen und sogar Singulettsauerstoff zu "quenchen", also durch einen physikochemischen Vorgang in den Triplettgrundzustand zu überführen. Stoffe mit dieser Eigenschaft werden auch als "Quenching Agents" bezeichnet.

Erfindungsgemäß ist daher auch die Verwendung der δ-Aminolävulinsäure, bzw. kosmetischer oder dermatologischer Zubereitungen mit einem wirksamen Gehalt an δ-Aminolävulinsäure bzw. der erfindungsgemäßen Kombinationen aus δ-Aminolävulinsäure sowie mindestens einer Substanz, gewählt aus der Gruppe der Antioxidantien, insbesondere der Tocopherole und Tocopherylester, als Radikalfänger, Antioxidans und/oder Quenching Agent für photochemisch erzeugte reaktive Substanzen wie Singulettsauerstoff.

Die erfindungsgemäßen kosmetischen oder dermatologischen Zubereitungen können auf an sich üblichen Formulierungsgrundlagen beruhen und zur Behandlung der Haut und/oder der Haare im Sinne einer dermatologischen Behandlung oder einer Behandlung im Sinne der pflegenden Kosmetik dienen. Sie enthalten vorteilhaft 0,01 bis 10 Gew.-%, insbesondere aber 0,1 bis 6 Gew.-%, bezogen auf das Gesamtgewicht, an δ-Aminolävulinsäure.

Die erfindungsgemäßen kosmetischen oder dermatologischen Zubereitungen enthalten bevorzugt 0,01 bis 10 Gew.-%, insbesondere aber 0,1 bis 6 Gew.-%, bezogen auf das Gesamtgewicht, an einem oder mehreren Stoffen aus der Gruppe der Antioxidantien.

Zur Anwendung wird die δ-Aminolävulinsäure in der für Kosmetika oder Dermatika üblichen Weise auf die Haut und/oder die Haare in ausreichender Menge aufgebracht.

Besonders bevorzugt sind solche kosmetische und dermatologische Zubereitungen, die in der Form eines Sonnenschutzmittels vorliegen. Vorteilhaft enthalten diese zusätzlich mindestens einen UVA-Filter und/oder mindestens einen UVB-Filter und/oder mindestens ein anorganisches Pigment.

Äußerst vorteilhaft sind aber auch solche Zubereitungen, welche nach der Lichtexposition auf die Haut aufgetragen werden, also Après-Soleil-Produkte. Es liegt bei solchen Zubereitungen im Ermessen des Fachmannes, ob zusätzliche UV-Filtersubstanzen verwendet werden sollen oder nicht.

Darüberhinaus werden Zubereitungen zur Prävention oder Bekämpfung der sogenannten Mallorca-Akne (Acne aestivalis) als vorteilhafte Verkörperungen der vorliegenden Erfindung angesehen.

Kosmetische Zubereitungen gemäß der Erfindung zum Schutze der Haut vor UV-Strahlen können in verschiedenen Formen vorliegen, wie sie z.B. üblicherweise für diesen Typ von Zubereitungen eingesetzt werden. So können sie z.B. eine wäßrige, alkoholische oder wäßrig alkoholische Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), ein Gel, eine Hydrodispersion, einen festen Stift oder auch ein Aerosol darstellen.

Die erfindungsgemäßen kosmetischen Zubereitungen können kosmetische Hilfsstoffe enthalten, wie sie üblicherweise in solchen Zubereitungen verwendet werden, z.B. Konservierungsmittel, Bakterizide, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Sofern die kosmetische oder dermatologische Zubereitung eine Lösung oder Lotion darstellt, können als Lösungsmittel verwendet werden:
- Wasser oder wäßrige Lösungen;
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Emulsionen gemäß der Erfindung z.B. in Form einer Sonnenschutzcreme, einer Sonnenschutzlotion oder einer Sonnenschutzmilch sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Insbesondere, wenn ein Produkt angestrebt ist, welches Prävention oder Linderung der Mallorca-Akne anstrebt, ist erfindungsgemäß vorteilhaft, die δ-Aminolävulinsäure oder die erfindungsgemäßen Wirkstoffkombinationen auf Basis der δ-Aminolävulinsäure in Hydrodispersionen oder Gele einzuarbeiten.

Kosmetische und dermatologische Zubereitungen zur Behandlung und Pflege der Haut können als Gele vorliegen, die neben δ-Aminolävulinsäure und dafür üblicherweise verwendeten Lösungsmitteln noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Erfindungsgemäße Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Hydrodispersionen stellen Dispersionen einer flüssigen, halbfesten oder festen inneren (diskontinuierlichen) Lipidphase in einer äußeren wäßrigen (kontinuierlichen) Phase dar.

Im Gegensatze zu O/W-Emulsionen, die sich durch eine ähnliche Phasenanordnung auszeichnen, sind Hydrodispersionen aber im wesentlichen frei von Emulgatoren. Hydrodispersionen stellen, wie im übrigen auch Emulsionen, metastabile Systeme dar und sind geneigt, in einen Zustand zweier in sich zusammenhängender diskreter Phasen überzugehen. In Emulsionen verhindert die Wahl eines geeigneten Emulgators die Phasentrennung.

Bei Hydrodispersionen einer flüssigen Lipidphase in einer äußeren wäßrigen Phase kann die die Stabilität eines solchen Systems beispielsweise dadurch gewährleistet werden, daß in der wäßrigen Phase ein Gelgerüst aufgebaut wird, in welchem die Lipidtröpfchen stabil suspendiert sind.

Feste Stifte gemäß der Erfindung können z.B. natürliche oder synthetische Wachse, Fettalkohole oder Fettsäureester enthalten. Bevorzugt werden Lippenpflegestifte.

Als Treibmittel für erfindungsgemäße, aus Aerosolbehältern versprühbare kosmetische oder dermatologische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Bevorzugt können die erfindungsgemäßen Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen. Sie können auch als Sonnenschutzmittel dienen.

Die UVB-Filter können öllöslich oder wasserlöslich sein. Als öllösliche Substanzen sind z.B. zu nennen:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin.

Als wasserlösliche Substanzen sind z.B. zu nennen:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Gegenstand der Erfindung ist auch die Kombination aus δ-Aminolävulinsäure mit einem oder mehreren UVB-Filtern bzw. erfindungsgemäße kosmetische oder dermatologische Zubereitungen, welche auch einen oder mehrere UVB-Filter enthalten.

Es kann auch von Vorteil sein, δ-Aminolävulinsäure mit UVA-Filtern zu kombinieren, die bisher üblicherweise in kosmetischen und/oder dermatologischen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Ferner werden vorteilhafte Zubereitungen erhalten, wenn δ-Aminolävulinsäure mit UVA- und UVB-Filtern kombiniert wird.

Auch Kombinationen aus δ-Aminolävulinsäure, einem oder mehreren Antioxidantien und einem oder mehren UVA-Filtern und/oder einem oder mehren UVB-Filtern sind erfindungsgemäß besonders vorteilhaft.

Kosmetische oder dermatologische Zubereitungen, δ-Aminolävulinsäure enthaltend, können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Auch die Kombinationen von
- δ-Aminolävulinsäure und anorganischen Pigmenten
- δ-Aminolävulinsäure und Antioxidantien und anorganischen Pigmenten
- δ-Aminolävulinsäure und UV-Filtersubstanzen und anorganischen Pigmenten
- δ-Aminolävulinsäure und Antioxidantien und UV-Filtersubstanzen und anorganischen Pigmenten
sind Gegenstand der Erfindung. Es können die für die vorstehenden Kombinationen genannten Mengen verwendet werden.

Gegenstand der Erfindung ist auch das Verfahren zur Herstellung der erfindungsgemäßen kosmetischen Zubereitungen, das dadurch gekennzeichnet ist, daß man in an sich bekannter Weise δ-Aminolävulinsäure in kosmetische oder dermatologische Formulierungen einarbeitet.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiel 1

| Sonnengel LF 4 (transparent) | |
|---|---|
| | Gew.-% |
| Benzophenon-4 | 0,5 |
| Phenylbenzimidazolsulfonsäure | 1,3 |
| δ-Aminolävulinsäure | 1,0 |
| Acrylamid/Natriumacrylat-Copolymer | 1,6 |
| Ethanol | 5,0 |
| Glycerin | 15,0 |
| NaOH (15-%ig) | q.s. |
| Parfum, Konservierungsmittel | q.s. |
| Wasser, VES | ad 100,0 |

### Beispiel 2

| Hydrodispersion LF 6 | |
|---|---|
| | Gew.-% |
| Octylmethoxycinnamat | 5,0 |
| Butylmethoxydibenzoylmethan | 1,0 |
| Phenyltrimethicon | 1,0 |
| Carbomer (Carbopol 981) | 1,0 |
| Hydroxypropylmethylcellulose | 0,2 |
| Butylenglycol | 3,0 |
| δ-Aminolävulinsäure | 0,5 |
| Tromethamin | q.s. |
| EDTA-Lösung (14-%ig) | 0,5 |
| Ethanol | 5,0 |
| Parfum, Konservierungsmittel | q.s. |
| Wasser, VES | ad 100,0 |

### Beispiel 3

| Sonnenmilch O/W | |
|---|---|
| | Gew.-% |
| Octylmethoxycinnamat | 5,0 |
| Butylmethoxydibenzoylmethan | 1,0 |
| Cetearylalkohol + PEG-40 Rizinusöl + Natriumcetearylsulfat | 2,5 |
| Glyceryllanolat | 1,0 |
| Laurylmethicon Copolyol | 0,5 |
| Mineralöl (DAB 9) | 5,0 |
| Caprylic/capric Triglyceride | 5,0 |
| Acrylamid/natriumacrylat Copolymer | 0,3 |
| Cyclomethicon | 2,0 |
| TiO₂ | 1,0 |
| δ-Aminolävulinsäure | 1,0 |
| Glycerin | 3,0 |
| EDTA-Lösung (14-%ig) | 0,5 |
| Ethanol | 5,0 |
| Parfum, Konservierungsmittel | q.s. |
| Wasser, VES | ad 100,0 |

### Beispiel 4

| Pflegelotion W/O | |
|---|---|
| | Gew.-% |
| Cyclomethicon | 3,0 |
| PEG-1-Glycerin Sorbitan Oleostearat | 1,7 |
| PEG-7 Hydriertes Rizinusöl | 6,3 |
| Mineralöl (DAB 9) | 13,0 |
| Caprylic/capric Triglyceride | 13,0 |
| δ-Aminolävulinsäure | 3,0 |
| Glycerin | 4,0 |
| MgSO₄ | 0,7 |
| Parfum, Konservierungsmittel | q.s. |
| Wasser, VES | ad 100,0 |

### Beispiel 5

| Pflegende Tagescrème O/W | |
|---|---|
| | Gew.-% |
| PEG-5 Glycerylstearat | 2,00 |
| Glycerylstearat | 3,00 |
| Cyclomethicon | 3,00 |
| Caprylic/capric Triglyceride | 3,00 |
| Cetylalkohl | 3,00 |
| Octylmethoxycinnamat | 2,50 |
| δ-Aminolävulinsäure | 5,00 |
| Ethanol | 1,00 |
| Hyaluronsäure | 0,05 |
| Tocopherylacetat | 0,50 |
| Glycerin | 4,00 |
| Parfum, Konservierungsmittel | q.s. |
| Wasser, VES | ad 100,00 |

### Beispiel 6

| Sonnencrème W/O | |
|---|---|
| | Gew.-% |
| PEG-22-Dodecyl Glycol Copolymer | 3,0 |
| Cetyl Dimethicon Copolyol | 2,0 |
| Cyclomethicon | 4,0 |
| Octylmethoxycinnamat | 7,0 |
| Methylbenzylidencampher | 3,4 |
| Butylmethoxydibenzoylmethan | 1,0 |
| Mineralöl (DAB 9) | 4,0 |
| Caprylic/capric Triglyceride | 4,0 |
| δ-Aminolävulinsäure | 2,5 |
| Glycerin | 4,00 |
| Parfum, Konservierungsmittel | q.s. |
| Wasser, VES | ad 100,00 |

### Beispiel 7

| After Sun Lotion | |
|---|---|
| | Gew.-% |
| Cetearylalkohol + PEG-40 Rizinusöl + Natriumcetearylsulfat | 2,50 |
| Glycerylstearat SE | 0,60 |
| Mineralöl (DAB 9) | 4,00 |
| Caprylic/capric Triglyceride | 2,00 |
| Schibutter | 2,00 |
| Avocadoöl | 2,00 |
| Tocopherylacetat | 3,00 |
| δ-Aminolävulinsäure | 2,00 |
| Acrylamid/natriumacrylat Copolymer | 0,30 |
| Glycerin | 4,00 |
| Hyaluronsäure | 0,05 |
| Bisabolol | 0,05 |
| Parfum, Konservierungsmittel | q.s. |
| Wasser, VES | ad 100,00 |

Im folgenden Versuch wird die vorteilhafte Wirkung der δ-Aminolävulinsäure demonstriert.

### Versuch:

Die antioxidative Wirkung von δ-Aminolävulinsäure mittels Chemilumineszenzmessung wurde ermittelt. Chemilumineszenz deutet auf oxidative Prozesse in der Haut, Minderung der Chemilumineszenz ist gleichbedeutend mit einer Herabsetzung oxidativer Prozesse.
- Testmaterial:: Acrylatgel mit 5 Gew.-% an δ-Aminolävulinsäure (ALA)
- Methode:: Messung der Chemilumineszenz der Hände vor und 3 Stunden nach Behandlung mit ALA bzw. Placebo (Gel ohne δ-Aminolävulinsäure)
- Probanden:: 12 Personen, 4 männlich, 8 weiblich.
- Testzeitraum:: 3 Tage.
- Applikation:: ALA wird volumetrisch abgemessen (90 µl) und mit Fingerling auf dem rechten bzw. linken Handrücken in einer 5,6 cm x 8 cm großen Fläche aufgetragen. Auf dem jeweils anderen Handrücken wird der PLacebo aufgetragen. Die Auftragungsorte (rechte Hand - linke Hand) werden zufällig gewählt.
- Testablauf:: 1. Tag
Markierung eines Areals an den Handrücken im Bereich zwischen Daumenwurzel und erstem Mittelhandknochen und Messung von Reflexionsspektren. Chemilumineszenzmessungen vor und nach einer 30-sekündigen UVA-Bestrahlung mittels UVA-Spot (Fa. Hönle). Behandlung mit d-Als und Placebo, jeweils 2 mg/cm². 3 Stunden später Wiederholung der Messungen.
2. und 3. Tag
Durchführung des obenbeschriebenen Testablaufes mit 4 weiteren Probanden.
Meßbedingungen am LS 50: Synchron-Modus, interval: 0 nm, Range: 300 - 500 nm, ex slit: 4 nm, em slit: 3,5 nm, scan speed: 500 nm/min, 4 % attenuator.
- Ergebnisse:: 3 Stunden nach der Behandlung zeigt sich an den mit ALA behandelten Händen eine Lumineszenzminderung um 11,8 % im Vergleich zur Ausgangssituation. An den mit Placebo behandelten Händen ergibt sich erwartungsgemäß praktisch keine Änderung.

## Patentansprüche

1. Verwendung von δ-Aminolävulinsäure zur Herstellung kosmetischer oder dermatologischer Zubereitungen zum Schutze der Haut gegen schädlichen Einfluß von ultraviolettem Licht.

2. Verwendung nach Anspruch 1 zum Schutze gegen Acne aestivalis (Mallorca-Akne).

3. Verwendung nach Anspruch 1 zum Schutze gegen Hautalterung.

4. Kosmetische oder dermatologische Zubereitungen, enthaltend Wirkstoffkombinationen aus δ-Aminolävulinsäure und Antioxidantien.

5. Zubereitungen nach Anspruch 3, dadurch gekennzeichnet, daß die Antioxidantien gewählt werden aus der Gruppe bestehend aus Tocopherolen und deren Derivaten, besonders α-Tocopherol bzw. α-Tocopherylestern, insbesondere α-Tocopherylacetat, ferner Sesamol, Gallensäurederivaten wie Methyl-, Ethyl-, Propyl-, Amyl-, Butyl- und Laurylgallat, dem Koniferylbenzoat des Benzoeharzes, Nordihydroguajakharzsäure, Nordihydroguajaretsäure, Butylhydroxyanisol, Butylhydroxytoluol, Ascorbinsäure, Citronensäure, Phosphorsäure, Lecithin, Trihydroxybutyrophenon, Carotinen, Vitamin-A und dessen Derivaten, insbesondere Retinylpalmitat, Ascorbinsäure, Ascorbylpalmitat, Dilaurylthiodipropionat, Distearylthiodipropionat, Monoisopropylcitrat, Thiodipropionsäure und EDTA sowie EDTA-Derivaten.

6. Kosmetische oder dermatologische Zubereitungen, enthaltend Wirkstoffkombinationen aus δ-Aminolävulinsäure und einer oder mehreren Substanzen, die UV-Strahlung im UVA- oder UVB-Bereich absorbieren.

7. Verwendung nach Anspruch 1 oder Zubereitungen nach einem der Ansprüche 4 - 6, dadurch gekennzeichnet, daß die Zubereitungen 0,01 Gew.-% bis 10 Gew.-%, insbesondere aber 0,1 Gew.-% bis 6 Gew.-%, bezogen auf das Gesamtgewicht an δ-Aminolävulinsäure enthalten.

8. Zubereitungen nach einem der Ansprüche 4 - 7, dadurch gekennzeichnet, daß sie 0,01 Gew.-% bis 10 Gew.-%, insbesondere aber 0,1 Gew.-% bis 6 Gew.-%, bezogen auf das Gesamtgewicht an einem Stoff, gewählt aus der Gruppe der Antioxidantien, enthalten.

9. Kosmetisches Verfahren zum Schutze der Haut vor UVA- und UVB-Strahlung, dadurch gekennzeichnet, daß man eine kosmetische Zubereitung, welche δ-Aminolävulinsäure enthält, in ausreichender Menge auf die Haut aufbringt.

## Claims

1. Use of δ-aminolaevulinic acid for the preparation of cosmetic or dermatological preparations for protecting the skin against the harmful effect of ultraviolet light.

2. Use according to Claim 1 for protecting against acne aestivalis (Mallorca acne).

3. Use according to Claim 1 for protecting against skin ageing.

4. Cosmetic or dermatological preparations comprising active ingredient combinations of δ-aminolaevulinic acid and antioxidants.

5. Preparations according to Claim 3, characterized in that the antioxidants are chosen from the group consisting of tocopherols and derivatives thereof, particularly α-tocopherol and α-tocopheryl esters, in particular α-tocopheryl acetate, and also sesame oil, gallic acid derivatives, such as methyl, ethyl, propyl, amyl, butyl and lauryl gallate, the coniferyl benzoate of benzoin, nordihydroguaiac acid, butylated hydroxyanisole, butylated hydroxytoluene, ascorbic acid, citric acid, phosphoric acid, lecithin, trihydroxybutyrophenone, carotenes, vitamin A and derivatives thereof, in particular retinyl palmitate, ascorbic acid, ascorbyl palmitate, dilauryl thiodipropionate, distearyl thiodipropionate, monoisopropyl citrate, thiodipropionic acid and EDTA and EDTA derivatives.

6. Cosmetic or dermatological preparations comprising active ingredient combinations of δ-aminolaevulinic acid and one or more substances which absorb UV radiation in the UVA or UVB region.

7. Use according to Claim 1 or preparations according to one of Claims 4-6, characterized in that the preparations comprise from 0.01% by weight to 10% by weight, but in particular from 0.1% by weight to 6% by weight, based on the total weight of δ-aminolaevulinic acid.

8. Preparations according to one of Claims 4-7, characterized in that they comprise from 0.01% by weight to 10% by weight, but in particular from 0.1% by weight to 6% by weight, based on the total weight, of a substance chosen from the group of antioxidants.

9. Cosmetic method for protecting the skin against UVA and UVB radiation, characterized in that a cosmetic preparation which comprises δ-aminolaevulinic acid is applied to the skin in a sufficient amount.

## Revendications

1. Utilisation de l'acide δ-aminolévulique en vue de la préparation de préparations cosmétiques ou dermatologiques pour protéger la peau contre l'influence nocive de la lumière ultraviolette.

2. Utilisation selon la revendication 1, pour protéger la peau vis-à-vis de l'acné aestivalis (acné de Majorque).

3. Utilisation selon la revendication 1, pour protéger la peau vis-à-vis du vieillissement de la peau.

4. Préparations cosmétiques ou dermatologiques, contenant des combinaisons de substances actives à base d'acide δ-aminolévulique et d'antioxydants.

5. Préparations selon la revendication 3, caractérisées en ce que les antioxydants sont choisis parmi le groupe constitué de tocophérols et de leurs dérivés, en particulier l'α-tocophérol ou des esters α-tocophéryliques, en particulier, l'acétate d'α-tocophéryle, et en outre du sésamol, de dérivés de l'acide gallique tels que le gallate de méthyle, d'éthyle, de propyle, d'amyle, de butyle et de lauryle, du coniférylbenzoate du benjoin, l'acide de résine de nordihydrogayac, de l'acide nordihydroguaiarétique, du butylhydroxyanisol, du butylhydroxytoluène, de l'acide ascorbique, de l'acide citrique, de l'acide phosphorique, de la lécithine, de la trihydroxybutyrophénone, de carotènes, de la vitamine A et de ses dérivés, en particulier le palmitate de rétinyle, du palmitate d'ascorbyle, du thiodipropionate de dilauryle, du thiodipropionate de distéaryle, du citrate de mono-isopropyle, de l'acide thiodipropionique et de l'EDTA ainsi que de dérivés de l'EDTA.

6. Préparations cosmétiques ou dermatologiques, contenant des combinaisons de substances actives à base d'acide δ-aminolévulique et d'une ou de plusieus substances qui absorbent le rayonnement UV dans le domaine 'UVA ou UVB.

7. Utilisation selon la revendication 1 ou préparations selon l'une quelconque des revendications 4-6, caractérisées en ce que les préparations contiennent de 0,01% en poids à 10% en poids, en particulier de 0,1% en poids à 6% en poids, d'acide δ-amino-lévulique par rapport au poids total.

8. Préparations selon l'une quelconque des revendications 4-7, caractérisées en ce qu'elles contiennent de 0,01% en poids à 10% en poids, en particulier de 0,1% en poids à 6% en poids, par rapport au poids total, d'une substance choisie parmi le groupe des antioxydants.

9. Procédé cosmétique pour la protection de la peau vis-à-vis du rayonnement UVA ou UVB, caractérisé en ce que l'on applique sur la peau, en quantité suffisante, une préparation cosmétique qui contient de l'acide lévulique.6
